# EUROPEAN PATENT SPECIFICATION

(11) **EP 0 745 332 B1**
(45) Date of publication and mention of the grant of the patent: **29.09.2004**
(21) Application number: 95201445.4
(22) Date of filing: 01.06.1995
(51) Int. Cl.: A23L 1/29, A23L 3/3463, A61K 35/78

(54) **Clinical nutritional composition**
Klinische Nährungszusammensetzung
Composition nutritive clinique

(43) Date of publication of application: 04.12.1996
(73) Proprietor: N.V. Nutricia, NL-2712 HM Zoetermeer (NL)
(72) Inventor: Goethals, Maud, 2611 AB Delft (NL)
(74) Representative: van Westenbrugge, Andries

(56) References cited:
- EP-A- 0 123 345
- DE-A- 3 433 722
- DE-A- 4 135 805
- FR-A- 2 201 043
- FR-A- 2 229 357
- GB-A- 873 476
- GB-A- 1 113 435
- US-A- 4 181 743
- US-A- 4 976 960
- DATABASE WPI Section Ch, Week 9221 Derwent Publications Ltd., London, GB; Class D12, AN 92-174149 & SU-A-1 658 977 (A MED KAZA SECT SUPPLY INST) , 30 June 1990
- DATABASE WPI Section Ch, Week 9214 Derwent Publications Ltd., London, GB; Class B05, AN 92-111641 & JP-A-04 058 847 (NIPPON OILS & FATS)
- DATABASE WPI Section Ch, Week 9015 Derwent Publications Ltd., London, GB; Class D13, AN 90-113338 & JP-A-02 065 757 (SAMUSAN)
- DATABASE WPI Section Ch, Week 8628 Derwent Publications Ltd., London, GB; Class B04, AN 86-178293 & JP-A-61 109 731 (FUJIKAWA)
- DATABASE WPI Section Ch, Week 8050 Derwent Publications Ltd., London, GB; Class B05, AN 80-89483c & SU-A-730 338 (A MED NUTRITION)
- PATENT ABSTRACTS OF JAPAN vol. 012, no. 166 (P-704) 19 May 1988 & JP 62 279 587 A (ALPS ELECTRIC CO LTD) 04 December 1987
- PATENT ABSTRACTS OF JAPAN vol. 013, no. 262 (C-608) 16 June 1989 & JP 01 063 364 A (SEIKEN KK) 09 March 1989
- PATENT ABSTRACTS OF JAPAN vol. 015, no. 259 (C-0846) 02 July 1991 & JP 03 087 159 A (TERUMO CORP) 11 April 1991
- PATENT ABSTRACTS OF JAPAN vol. 010, no. 355 (C-388) 29 November 1986 & JP 61 155 322 A (RIKEN KAGAKU KOGYO KK) 15 July 1986
- DATABASE WPI Week 199350, Derwent Publications Ltd., London, GB; Class D13, AN 1993-395867 & CN 1 068 714 A (HUNAN INST BIOLOGY) 10 February 1993
- DATABASE WPI Week 197810, Derwent Publications Ltd., London, GB; Class A96, AN 1978-18747A & JP 53 009 309 A (MSC YG) 27 January 1978
- HERTOG M.G.L. ET AL THE LANCET vol. 342, 23 October 1993, pages 1007 - 1011
- SAINANI G.S. ET AL INDIAN J MED RES vol. 69, 1979, pages 776 - 780

## Description

The invention relates to clinical nutritional compositions which can be administered under conditions where ingesting solid food in a normal way is hampered, e.g. in pre- or post-operational conditions or inflammatory conditions or during organ injuries. These compositions may be ingested through the normal tract, but in severe situations, they are often administered by means of a tube or similar means, which can be conducted through the nose, throat, directly into the stomach or into the intestines. In exceptional cases they may also be introduced through the skin into the stomach or into the intestine, by means of a catheter. These nutritional compositions are sometimes referred to as enteral clinical nutrition (ECN), nasogastric foods or tube feeding. Depending on the particular use, they contain the entire balanced daily diet, either in a homogeneous, essentially liquid form, or in powder form, or they contain parts of a diet to be fortified and are thus used as a food supplement.

DE-A-4135805 discloses a mixture of ground or pressed garlic bulbs and honey which has been ripened for at least 4 weeks and which has health promoting effects on the respiratory tract, the gastro-intestinal and the cardiovascular system. DE-A-3433722 describes a mixture containing garlic paste, lemon juice, garlic and salt which is claimed to have various health effects. FR-A-2201043 discloses a nutritional composition containing an aged alcoholic extract of garlic and herbs, which improves vitality. SU-A-1658977 describes a dietetic product containing 5% of onion, 50% of fish filets and further components for the treatment of adiposity. According to JP-A-2-65757, deodorised garlic extract is useful for improving physical fitness. The use of onion powder in food for treating dysfunctional bleeding is described in JP-A-61-109731. SU-A-730338 discloses a tube feeding composition containing a vegetable mixture including 0.3-0.4 % by weight of onion.

The currently available nutritional compositions of this type have a number of drawbacks, which are apparently due to an insufficient balance between the various essential nutritional components of the diet. These imbalances can lead to loss of weight of the patient, diarrhoea and retarded recovery of a diseased state.

Thus there is a need for improved nutritional compositions for administration in essentially liquid form. It has been found now that components of *Allium* species such as onion can be used as a valuable ingredient in such nutritional compositions, primarily to improve the nutritional value of the compositions, but also to stabilise other nutritional components of the compositions.

Accordingly, the invention concerns the use of material derived from the bulb of *Allium* species for the manufacture of clinical nutrition or infant nutrition for feeding patients suffering from conditions where ingestion of solid food in a normal way is hampered, said clinical or infant nutrition being a complete nutritional composition in essentially liquid form suitable for enteral administration, and comprising:
a. 40-78 en% carbohydrates;
b. 4-25 en% proteinaceous material;
c. 8-50 en% fat; and
d. material derived from the bulb of *Allium* species in an amount obtained from 10-70 g of the fresh *Allium* product per liter of the complete nutritional composition.

The invention also concerns a clinical nutrition or infant formula composition in essentially liquid form suitable for enteral administration comprising:
a. 40-78 en% carbohydrates;
b. 4-25 en% proteinaceous material;
c. 8-50 en% fats; and
e. material derived from the bulb of *Allium* species in an amount obtained from 10-35 g of the fresh *Allium* product per liter of the complete nutritional composition, calculated on the basis of fresh *Allium* product.

The genus *Allium* is a member of the *Alliaceae* family, and comprises the common onion (*A. cepa*), wild onion (*A. cernuum*), garlic (*A. sativum*), wild garlic (*A. ursinum*), leek (*A. porrum*), chives (*A. schoenoprasum*) and shallot (*A. ascallonicum*). Parts, homogenates and extracts of these plants, especially of their corms, bulbs or stems, can be used in the compositions according to the invention.

Preference is given to the bulbs of the common onion, especially onion bulbs without their outer skin and roots. As an example, fresh onions contain on average, per 100 g of fresh material, 87.6 g of water, 1.25 g of proteins, 0.25 g of fats, 4.9 g of available carbohydrates (mainly sucrose, fructose and glucose), 1.8 g of fibres, 0.2 g of organic acids and 0.6 g of minerals. Thus an amount of *Allium*-derived material calculated on the basis of fresh *Allium* product, means for onions (100-87.6)/100 = about 1/8 of that amount of water-free material.

The material derived from the *Allium* species to be used in the nutritional compositions can be obtained in various ways. Firstly, the *Allium* material can be obtained by pressing the starting material, especially the bulb, which results in an aqueous (water-soluble) fraction which does not contain most of the cellular material. This aqueous phase can be used as such. Secondly, the volatiles that are poorly water-soluble can be obtained e.g. by steam distillation; this results in an essential oil, which can also be used as such. Thirdly the *Allium* material may be chopped or otherwise reduced in size and treated with hydrolytic enzymes such as cellulase or hemicellulase, causing the cell walls to be broken and their contents become available. Alternatively, the solid material remaining after the liquefaction step may be hydrolysed. Either of these hydrolysates can then be filtered and the filtrate used. Preferably, at least two of these fractions are used, or the entire hydrolysate is used in the nutritional compositions. Preferably, the *Allium*-derived material is not subjected to a heat treatment.

The amounts of *Allium*-derived material is calculated on the basis of fresh *Allium* product, i.e. on the amount of starting *Allium* material, without the parts that are removed (skin, roots). For example, if an onion extract is used, the amount of it to be incorporated in the nutritional composition, 1.4-140 g of fresh, peeled, derooted onions is used for each daily dosage of nutritional composition, and is processed to an extract and then added to the other constituents of the composition.

In the specification and claims, the amounts to be used are based on the daily nutritional requirement. It is assumed that, in case of a liquid composition representing a complete food, the daily need of the nutritional composition is 2 litres of liquid. If the required daily food intake should be higher or lower, the figures can be adapted accordingly. Thus a preferred concentration of *Allium*-derived material is 10-70, preferably 10-50, most preferably 10-35 g, calculated on the basis of fresh *Allium* product, per litre of liquid complete nutritional composition.

The nutritional compositions according to the invention also comprise carbohydrates , fats and proteinaceous material.

The carbohydrates constitute the preferred energy source in most cases. Carbohydrates to be used may include monosaccharides such as glucose, fructose, galactose and ribose, disaccharides such as lactose, sucrose, lactulose and maltose, and oligo- and polysaccharides such as starch-derived or inulin type material, including maltodextrines. The latter are the preferred carbohydrates for nutritional compositions intended for clinical nutrition.

The proteins to be used in the nutritional compositions according to the invention can be those commonly present in tube feeding, infant formulae and other foodstuffs. Preferred proteinaceous materials to be used according to the invention include partially denatured whey proteins, or partially hydrolysed whey proteins. The partly denatured whey proteins are especially beneficial for complete nutritional compositions. The preferred level of partly denatured whey proteins corresponds to at least 20 % of the total protein content, more preferably 40-100 wt.% of the total protein content, or alternatively preferably 2-30, more preferably 4-20 wt.% of the dry matter content of the composition.

For clinical foods, especially for food supplements, proteinaceous material having an increased level of glutamine and/or cysteine, and sometimes also arginine, are preferred. For example, normal glutamine, arginine and cysteine levels are 17-26, 2.3-7.9 and 0.2-2.4 g per 16 g N, respectively, whereas these levels may be up to 43, up to 15 and up to 5 g/16 g N, respectively, in case of clinical food supplements.

Low-protein of protein-free compositions containing carbohydrates and *Allium*-derived material, and optionally fats, minerals, vitamins etc. can be prepared for patients having a low protein tolerance.

The fats contained in the nutritional compositions may be the usual nutritional fats, preferably containing unsaturated fatty acid residues, more preferably also polyunsaturated fatty acid residues (PUFA), such as linoleic and linolenic acid residues, and higher analogues. The preferred level of PUFA's (fatty acid residues ≥ 18) is at least 1.5 wt.% for infant formulae and at least 5 wt.% for clinical nutritional compositions, calculated on the total level of fatty acid residues. More preferred levels are at least 5 wt.% and at least 10 wt.% for infant formulae and clinical compositions respectively. The upper limits may be e.g. 20 and 50 wt.%, respectively. The preferred level of LC-PUFA's (fatty acid residues ≥ 20) is at least 0.3 wt.% for infant formulae and at least 0.5 wt.% for clinical nutritional compositions. More preferred levels are at least 0.5 and at least 1.0, respectively, up to e.g. 4 wt.% for infant formulae and 6 wt.% for clinical compositions.

The relative proportion of carbohydrates, proteins and fats in complete nutritional compositions according to the invention may be (in energy %) 40-78, 4-25 and 8-50 respectively, which corresponds to about 50-90 wt.% carbohydrates, 5-30 wt.% proteins and 4-25 wt.% fats.

In addition, the nutritional compositions will usually contain sodium, potassium, calcium, magnesium, chloride and phosphate ions in the range of tens or hundreds of mg per 100 g of dry product, and furthermore trace elements, like iron, zinc, copper, manganese (in the 100 µg - 1 mg /100 g range) and fluorine, molybdenum, selenium, chromium and iodine (in the µg/100 g range). Other preferred components include vitamins (A, D, E, K; B6, B12, C), thiamine, riboflavine, niacin, pantothenic acid, folic acid, biotin, choline and myoinositol. Also, antioxidants, such as β-carotene, tocopherols, lecithin and ascorbic acid (vitamin C) may advantageously be present in the compositions according to the invention.

It was found that the presence of antioxidants, especially flavonoids such as quercetin, is beneficial to the acceptance of the composition and to the recovery of the patient using the composition. Preferably the amount of such flavonoids (e.g. quercetin) in the nutritional composition is 0.5-50 mg, more preferably 5-25 mg, per day, or, in case of liquid compositions, per 2 litres.

The presence of the *Allium*-derived material does not only improve the nutritional or pharmacological value of the nutritional composition as such, but also stabilises other food ingredients, such as fats, proteins and vitamins. Thus other stabilisers, preservatives or antioxidants normally used in such compositions can be omitted or their level can be reduced. If the composition contains appreciable amounts of particularly labile components, such as polyunsaturated fatty acid residues, it may be recommendable to proportionally increase the level of *Allium*-derived material above the level required for nutritional purposes.

The following examples are only illustrative and do not fall within the scope of the invention.

An example of a dry composition which can be reconstituted by addition of water, is an infant formula. Such a formula may contain e.g. 10-12.5 g of protein, 50-65 g of carbohydrate (such as lactose), 25-35 g of fat, 0.5-8 g, especially 1-4 g of *Allium*-derived material, in addition to Na (145 mg), K (520 mg), Cl (320 mg), Ca (430 mg), P (220 mg) and Mg (40 mg) per 100 g, and further vitamins and trace elements according to the WHO or IDACE recommendations (Recommended Daily Allowances). About 12-15 g of this dry matter may be reconstituted to 100 ml of liquid infant feeding. The daily requirement of such liquid composition may e.g. be 600 mls.

Another example of a dry composition, which can be reconstituted to a low-fat, low-sodium liquid supplement, contains, per 100 g, 50-70 g of protein, preferably milk protein, 25-35 g of carbohydrate, e.g. lactose, 0.5-1 g of fat, in addition to Na (30 mg), K (1000 mg), Cl (500 mg), Ca (1700 mg), P (1200 mg) and Mg (120 mg) per 100 g, and further vitamins and trace elements, and further 2-20 g of *Allium*-derived material.

The nutritional compositions and supplements according to the invention can be used as main food for particular users such as infants, or as a clinical food for preoperational or postoperational conditions, or for the treatment of cancer, immune diseases, cardiac and vascular diseases, premature ageing or radical damage.

## Claims

1. Use of material derived from the bulb of *Allium* species for the manufacture of clinical nutrition or infant nutrition for feeding patients suffering from conditions where ingestion of solid food in a normal way is hampered, said clinical or infant nutrition being a complete nutritional composition in essentially liquid form suitable for enteral administration, and comprising:
a. 40-78 energy % carbohydrates;
b. 4-25 energy % proteinaceous material;
c. 8-50 energy % fat; and
d. material derived from the bulb of *Allium* species in an amount obtained from 10-70 g of the fresh *Allium* product per liter of the complete nutritional composition.

2. Use according to claim 1, wherein *Allium* species is *Allium cepa*.

3. Use according to claim 1, wherein *Allium* species is *Allium sativum*.

4. Use according to any one of claims 1-3, wherein the nutritional composition comprises material obtained from 10-35 g of the fresh *Allium* product per liter.

5. Use according to any one of claims 1-4, for the manufacture of a nutritional composition for the treatment of immune diseases, cardiac and vascular diseases, premature ageing or radical damage or for the recovery from heavy physical strain.

6. Use according to any one of claims 1-5, wherein the material derived from the bulb of *Allium* is a water-soluble fraction or hydrolysate of the bulb *of Allium.*

7. Use according to any one of claims 1-6, wherein the fats provide at least 1.5 wt.% polyunsaturated fatty acid residues for infant formula or at least 5 wt.% polyunsaturated fatty acid residues for clinical nutrition, calculated on the total level of fatty acid residues.

8. Use according to any one of claims 1-7, wherein the carbohydrates comprises monosaccharides, disaccharides, oligosaccharides and polysaccharides.

9. Clinical nutrition or infant formula composition in essentially liquid form suitable for enteral administration comprising:
a. 40-78 energy % carbohydrates;
b. 4-25 energy % proteinaceous material;
c. 8-50 energy % fats; and
e. material derived from the bulb of *Allium* species in an amount obtained from 10-35 g of the fresh *Allium* product per liter of the complete nutritional composition, calculated on the basis of fresh *Allium* product.

10. Composition according to claim 9, wherein the carbohydrate comprises monosaccharides, disaccharides, oligosaccharides and polysaccharides.

11. Composition according to claim 9 or 10, wherein the protein comprises partially denatured whey protein or partially hydrolysed whey protein.

12. Composition according to any one of claims 9-11, wherein the fat provides at least 1.5 wt.% polyunsaturated fatty acid residues for infant formula and at least 5 wt.% polyunsaturated fatty acid residues for clinical nutrition, calculated on the total level of fatty acid residues.

13. Composition according to any one of claims 9-12, wherein said material derived from *Allium* species is a water soluble fraction, an extract or hydrolysate of the bulb of the *Allium* species.

## Patentansprüche

1. Verwendung eines Materials, abgeleitet von der Zwiebel von Alliumarten für die Herstellung einer klinischen Ernährung oder Kleinkinderernährung für die Ernährung von Patienten, die an Zuständen leiden, bei denen die Verdauung von festen Nahrungsmitteln auf normalem Wege behindert ist, wobei diese klinische oder Kleinkindernährung eine vollständige Ernährungszusammensetzung in im wesentlichen flüssiger Form ist, die für die enterale Verabreichung geeignet ist und umfassend:
a) 40 bis 78 Energie% Kohlenhydrate;
b) 4 bis 25 Energie% proteinartige Materialien;
c) 8 bis 50 Energie% Fett und
d) Material, abgeleitet von der Zwiebel von Alliumarten in einer Menge, erhalten aus 10 bis 70 g des frischen Alliumprodukts pro Liter der vollständigen Ernährungszusammensetzung.

2. Verwendung gemäß Anspruch 1, worin die Alliumart Allium cepa ist.

3. Verwendung gemäß Anspruch 1, worin die Alliumart Allium sativum ist.

4. Verwendung gemäß einem der Ansprüche 1 bis 3, worin die Ernährungszusammensetzung Material umfasst, erhalten von 10 bis 35 g eines frischen Alliumprodukts pro Liter.

5. Verwendung gemäß einem der Ansprüche 1 bis 4 für die Herstellung einer Ernährungszusammensetzung für die Behandlung von Immunerkrankungen, Herz- und Gefäßerkrankungen, vorzeitiger Alterung oder Radikalschäden oder für die Erholung nach schwerer physischer Belastung.

6. Verwendung gemäß einem der Ansprüche 1 bis 5, worin das Material, abgeleitet von der Zwiebel von Allium, eine wasserlösliche Fraktion oder ein Hydrolysat der Zwiebel von Allium ist.

7. Verwendung gemäß einem der Ansprüche 1 bis 6, worin die Fette mindestens 1,5 Gew.% polyungesättigte Fettsäurereste für eine Kleinkinderformel oder mindestens 5 Gew.% polyungesättigte Fettsäurereste für die klinische Ernährung bereitstellen, berechnet am Gesamtniveau der Fettsäurereste.

8. Verwendung gemäß einem der Ansprüche 1 bis 7, worin die Kohlenhydrate Monosaccharide, Disaccharide, Oligosaccharide und Polysaccharide umfassen.

9. Klinische Ernährungs- oder Kleinkinderformelzusammensetzung in im wesentlichen flüssiger Form, die für die enterale Verabreichung geeignet ist, umfassend:
a) 40 bis 78 Energie% Kohlenhydrate;
b) 4 bis 25 Energie% proteinartige Materialien;
c) 8 bis 50 Energie% Fette und
e) Material, abgeleitet von der Zwiebel von Alliumarten in einer Menge, erhalten aus 10 bis 35 g des frischen Alliumprodukts pro Liter der vollständigen Ernährungszusammensetzung, berechnet auf der Basis des frischen Alliumprodukts.

10. Zusammensetzung gemäß Anspruch 9, worin die Kohlenhydrate Monosaccharide, Disaccharide, Oligosaccharide und Polysaccharide umfassen.

11. Zusammensetzung gemäß Anspruch 9 oder 10, worin das Protein teilweise denaturiertes Molkeprotein oder teilweise hydrolysiertes Molkeprotein umfasst.

12. Zusammensetzung gemäß einem der Ansprüche 9 bis 11, worin die Fette mindestens 1,5 Gew.% polyungesättigte Fettsäurereste für eine Kleinkinderformel und mindestens 5 Gew.% polyungesättigte Fettsäurereste für die klinische Ernährung bereitstellen, berechnet am Gesamtniveau der Fettsäurereste.

13. Zusammensetzung gemäß einem der Ansprüche 9 bis 12, worin das Material, abgeleitet von Alliumarten, eine wasserlösliche Fraktion, ein Extrakt oder ein Hydrolysat der Zwiebel der Alliumart ist.

## Revendications

1. Utilisation de matière dérivée du bulbe d'espèces d'*Allium* pour la préparation de nutriment clinique ou de nutriment infantile pour nourrir des patients souffrant de maladies où l'ingestion d'un aliment solide de façon normale est empêchée, ladite alimentation clinique ou alimentation infantile étant une composition nutritionnelle complète sous forme essentiellement liquide, appropriée pour une administration entérique, et comprenant :
a. 40-78% en énergie de glucides;
b. 4-25% en énergie de matière protéinée ;
c. 8-50% en énergie de graisses, et
d. de la matière dérivée du bulbe d'espèces d'*Allium* dans une quantité obtenue à partir de 10-70 g de produit d'*Allium* frais par litre de composition nutritionnelle complète.

2. Utilisation selon la revendication 1, dans laquelle l'espèce d'*Allium* est *Allium cepa*.

3. Utilisation selon la revendication 1, dans laquelle l'espèce d'*Allium* est *Allium sativum*.

4. Utilisation selon l'une quelconque des revendications 1 à 3, dans laquelle la composition nutritionnelle comprend de la matière obtenue à partir de 10-35 g de produit d'*Allium* frais par litre.

5. Utilisation selon l'une quelconque des revendications 1 à 4, pour la fabrication d'une composition nutritionnelle destinée au traitement de maladies immunitaires, de maladies cardiaques et vasculaires, de vieillissement prématuré ou de dommages radicaux, ou pour la récupération après un effort physique intense.

6. Utilisation selon l'une quelconque des revendications 1 à 5, dans laquelle la matière dérivée du bulbe d'*Allium* est une fraction hydrosoluble ou un hydrolysat du bulbe d'*Allium.*

7. Utilisation selon l'une quelconque des revendications 1 à 6, dans laquelle les graisses fournissent au moins 1,5% en poids de résidus d'acides gras polyinsaturés pour la formulation infantile ou au moins 5% en poids de résidus d'acides gras polyinsaturés pour le nutriment clinique, calculés sur le taux total des résidus d'acides gras.

8. Utilisation selon l'une quelconque des revendications 1 à 7, dans laquelle les glucides comprennent des monosaccharides, des disaccharides, des oligosaccharides et des polysaccharides.

9. Composition nutritionnelle clinique ou infantile sous forme essentiellement liquide, appropriée pour une administration entérique, et comprenant :
a. 40-78% en énergie de glucides;
b. 4-25% en énergie de matière protéinée ;
c. 8-50% en énergie de graisses, et
d. de la matière dérivée du bulbe d'espèces d'*Allium* dans une quantité obtenue à partir de 10-35 g de produit d'*Allium* frais par litre de composition nutritionnelle complète, calculée sur la base du produit d'*Allium* frais.

10. Composition selon la revendication 9, dans laquelle les sucres comprennent des monosaccharides, des disaccharides, des oligosaccharides et des polysaccharides.

11. Composition selon la revendication 9 ou 10, dans laquelle la protéine comprend la protéine lactosérique partiellement dénaturée ou la protéine lactosérique partiellement hydrolysée.

12. Composition selon l'une quelconque des revendications 9 à 11, dans laquelle les graisses fournissent au moins 1,5% en poids de résidus d'acides gras polyinsaturés pour la formulation infantile on au moins 5% en poids de résidus d'acides gras polyinsaturés pour le nutriment clinique, calculés sur le taux total des résidus d'acides gras.

13. Composition selon l'une quelconque des revendications 9 à 12, dans laquelle ladite matière dérivée de l'espèce d'*Allium* est une fraction hydrosoluble, un extrait ou un hydrolysat du bulbe de l'espèce d'*Allium*.
